## Europäisches Patentamt
## European Patent Office
## Office européen des brevets

(19)

(11) Veröffentlichungsnummer: **0 000 486**
**B1**

(12) **EUROPÄISCHE PATENTSCHRIFT**

(45) Veröffentlichungstag der Patentschrift: **29.07.81**

(21) Anmeldenummer: **78100312.4**

(22) Anmeldetag: **06.07.78**

(51) Int. Cl.³: **C 07 G 7/00, C 12 N 11/10**

(54) Verfahren zur Herstellung von stabilisierten trägergebundenen Proteinen.

(30) Priorität: **16.07.77 DE 2732301**

(43) Veröffentlichungstag der Anmeldung:
**07.02.79 Patentblatt 79/3**

(45) Bekanntmachung des Hinweises auf die Patenterteilung:
**29.07.81 Patentblatt 81/30**

(84) Benannte Vertragsstaaten:
**CH DE FR GB NL SE**

(56) Entgegenhaltungen:
**DE - A - 2 215 509**
**DE - A - 2 523 793**

(73) Patentinhaber: **Röhm GmbH**
**Kirschenallee**
**D-6100 Darmstadt (DE)**

(72) Erfinder: **Krämer, Dieter, Dr.**
**Rilke-Allee 153**
**D-6500 Mainz 31 (DE)**
Erfinder: **Sauber, Klaus, Dr.**
**Freiligrathstrasse 32**
**D-6232 Bad Soden (DE)**

# 0 000 486

Verfahren zur Herstellung von stabilisierten trägergebundenen Proteinen

Die Erfindung betrifft ein Verfahren zur Herstellung von stabilisierten trägergebundenen Proteinen. Bei Verfahren, die auf der biospezifischen Wechselwirkung zwischen einem Protein und einem korrespondierenden Substrat beruhen, werden die Proteine mit Vorteil in Form eines an einen festen Träger gebundenen Materials eingesetzt. Das Protein läßt sich dadurch durch einfaches Filtrieren von der behandelten Substratlösung abtrennen. Trägergebundene Proteine haben Bedeutung bei der Affinitätschromatographie und besonders bei der Durchführung enzymatischer Prozesse, wobei das von dem Träger gebundene Protein ein Enzym ist.

Gegenüber den freien Proteinen in wäßriger Lösung zeichnen sich trägergebundene Proteine in der Regel durch eine erhöhte Stabilität gegen Inaktivierung durch pH-Wert-Verschiebung, hohe Temperaturen oder Autoxydation aus. Trotzdem sinkt die biospezifische Wirksamkeit, z.B. im Falle von Enzymen die enzymatische Aktivität, bei mehrfacher Wiederverwendung des trägergebundenen Proteins bei jeder Verwendungsstufe etwas ab. Es ist bekannt, daß man den Aktivitätsverlust von Proteinen aufhalten oder rückgängig machen kann, wen man sie mit Merkaptanen behandelt (vgl. Dissertation Jan Carlsson, Uppsala 1974, S. 8, Abstract). Die Wirkung der Merkaptane wird darauf zurückgeführt, daß sie Disulfidbrücken innerhalb des Proteinmoleküls aufspalten, die durch Autoxydation aus Thiolgruppen entstanden sind. Diese Autoxydation wird als die Ursache des reversiblen Aktivitätsverlustes angesehen.

Es ist in den meisten Fällen jedoch nicht erwünscht, zur Aufrechterhaltung der Aktivität des gebundenen Proteins der Substratlösung ein Merkaptan zuzusetzen, weil die Substratlösung dadurch verunreinigt wird und eine zusätzliche aufwendige Reinigungsstufe erforderlich wird. Auch die getrennte Reaktivierung des trägergebundenen Proteins zwischen zwei Anwendungen ist wegen des zusätzlichen Arbeitsaufwandes keine befriedigende Lösung. Außerdem sind die meisten Mercaptane toxisch und übelriechend.

Aus der DE - OS 22 15 509 ist es bekannt, ein Enzym an einen Träger, der Anhydridgruppen als gegenüber Enzymen bindungsaktive Gruppen enthält, in einer wäßrigen Lösung in Gegenwart bekannter SH-Reagentien als Stabilisatoren zu binden. Ob damit eine dauerhaft erhöhte Stabilität des gebundenen Enzyms erreicht wird, ist nicht erwiesen. Die Bindungskapazität gegenüber Proteinen wird jedoch herabgesetzt, da die bindungsaktiven Gruppen bevorzugt mit den niedermolekularen SH-Verbindungen reagieren, wodurch vor allem die sterisch gut zugänglichen, gegenüber den großen Proteinmolekülen bindungsaktiven Gruppen schnell verbraucht werden.

Die Aufgabe der vorliegenden Erfindung bestand darin, trägergebundene Proteine mit erhöhter Stabilität gegen allmählichen Aktivitätsverlust in wäßriger Lösung durch Umsetzung des Proteins mit einem wasserunlöslichen Trägermaterial herzustellen. Die Aufgabe wird erfindungsgemäß dadurch gelöst, daß man ein merkaptogruppenfreies Trägermaterial einsetzt, das als bindungsaktive Gruppen Oxirangruppen enthält, und daß man auf das an den Träger gebundene Protein Schwefelwasserstoff oder eine Verbindung mit einem Molekulargewicht unter 5000 mit wenigstens einer Merkaptogruppe und wenigstens einer weiteren Merkapto-, Hydroxy- oder primären oder sekundären Aminogruppe einwirken läßt.

Die gegenüber dem Protein bindungsaktiven, sterisch gut zugänglichen Oxirangruppen stehen bei dem erfindungsgemäßen Verfahren vollständig zur Bindung des Proteins zur Verfügung, während die sterisch behinderten, für die großen Proteinmoleküle unzugänglichen Oxirangruppen nach der Bindung des Enzyms noch vorhanden und zur Umsetzung mit den verhältnismäßig kleinen Molekülen der Schwefelverbindung befähigt sind.

Da in der Fachwelt die Meinung vertreten wurde, daß die stabilisierende Wirkung von Merkaptanen auf ihrer Reaktion mit Disulfidbrücken beruht, war nicht zu erwarten, daß sie auch dann wirksam sein würden, wenn sie an das Trägermaterial gebunden und daher nicht in der Lage sind, in räumliche Nähe der Disulfidbrücken zu gelangen. Überraschenderweise wird durch die gebundenen Merkaptogruppen dennoch eine erhebliche Stabilisierung erreicht. In der nachfolgenden Tabelle wird am Beispiel einer trägergebundenen.

Penicillin-Acylase der Aktivitätsverlust bei mehrfacher Wiederverwendung mit und ohne Merkaptogruppen am Träger gegenübergestellt. Die Merkaptogruppen wurden in diesem Falle durch Bindung von Dithioerythrit an einen Epoxydgruppen enthaltenden Enzymträger eingeführt. Das Trägermaterial war ein vernetztes Acrylamidpolymerisat mit Epoxydgruppen, das zunächst mit Penicillin-Acylase und danach gegebenenfalls mit 50 bzw. 500 mg Dithioerythrit je Gramm des feuchten Trägermaterials umgesetzt worden war. Die Aktivität des Präparats gegenüber 6-Amino-penicillinsäure wurden in wiederholter Umsetzung von 1 bis 2 g des Präparats mit jeweils 5% Substrat in 200 ml Substratlösung (0,01 M Phosphatpuffer, pH 7,5) bei 37°C durch automatische Titration mit 1,0 N NaOH ermittelt. Die Ausgangsaktivität bei der ersten Verwendung wurde jeweils als 100% bezeichnet.

| Verwendungen | Zahl d. Aktivität in % der Ausgangsaktivität | | |
|---|---|---|---|
| | ohne Merkaptan | mit 50 mg Dithioerythrit | mit 500 mg Dithioerythrit |
| 1 | 100 | 100 | 100 |
| 2 | 96 | 100 | 100 |
| 3 | 89 | 99 | 98 |
| 4 | 85 | 96 | 98 |
| 5 | 76 | 96 | 98 |
| 6 | 75 | 96 | 96 |
| 7 | 69 | 97 | 95 |
| 8 | 65 | 96 | 92 |
| 9 | 64 | 96 | 95 |
| 10 | 64 | — | 95 |
| 11 | 61 | 84 | 95 |
| 12 | 56 | 79 | 93 |
| 15 | 52 | 75 | 93 |
| 20 | 43 | 65 | 89 |
| 25 | 34 | 58 | 75 |

Die erfindungsgemäß erreichte Stabilisierung beruht auf der Bindung von Schwefelwasserstoff oder einer wenigstens eine Merkaptogruppe und wenigstens eine weitere Merkapto-, Hydroxy- oder primäre oder sekundäre Aminogruppe enthaltenden Verbindung an die bindungsaktiven Oxirangruppen des Trägermaterials. Die Merkapto-, Hydroxy- oder Aminogruppen der genannten Verbindungen reagieren ebenso wie die entsprechenden Gruppen der zu bindenden Proteine mit den Oxirangruppen des Trägermaterials. Da die Verbindungen ein Molekulargewicht unter 5000 haben, vermögen sie aufgrund ihrer geringen Molekülgröße noch mit den Oxirangruppen des Trägermaterials zu reagieren, die für Proteinmoleküle aus Gründen sterischer Hinderung nicht zugänglich sind. Die Oxirangruppen reagieren mit Schwefelwasserstoff bzw. mit Merkapto-, Hydroxy- und Aminogruppen leichter als mit Wasser, so daß es möglich ist, sowohl die Umsetzung der Oxirangruppen mit dem Protein als auch die nachfolgende Umsetzung mit der Merkapto- und gegebenenfalls Hydroxy- oder Aminogruppen enthaltenden Verbindung in wäßriger Phase vorzunehmen. Da die Oxirangruppen dennoch allmählich durch Wasser zersetzt werden, sollte zwischen der ersten und der zweiten Umsetzungsstufe des erfindungsgemäßen Verfahrens kein unnötig langer Zeitraum liegen.

Die erwünschte Stabilisierungswirkung kommt dann zustande, wenn wenigstens eine Merkaptogruppe je gebundenes Molekül in freier Form erhalten bleibt und die weitere Merkapto-, Hydroxy- oder Aminogruppe mit der Oxirangruppe des Trägers reagiert. Da der Träger aus einer unlöslichen festen Matrix besteht und eine entsprechend niedrige Molekülbeweglichkeit hat, wird die Merkaptogruppenhaltige Verbindung zum weit überwiegenden teil nur mit einer ihrer funktionellen Gruppen an den Träger gebunden. Wenn diese Gruppe eine Hydroxyl- oder Aminogruppe ist, bleibt auf jeden Fall eine Merkaptogruppe erhalten. Bei Verwendung einer Verbindung mit nur einer Merkaptogruppe und einer oder mehreren Amino- oder Hydroxygruppen kann gerade die Merkaptogruppe mit der Oxirangruppe des Trägers reagieren, so daß nur noch Amino- oder Hydroxygruppen übrigbleiben. In diesem Fall trägt das gebundene Molekül nicht zur Stabiliserung bei. Da jedoch ein Teil der Moleküle nicht mit der Merkaptosondern mit einer Amino- oder Hydroxygruppe reagiert, bleiben im statistischen Mittel genügend stabilisierend wirkende Merkaptogruppen übrig.

Die Menge der Merkaptogruppen-haltigen Verbindung wird im allgemeinen so bemessen, daß wenigstens 0,3 Gew.-%, bezogen auf das Feuchtgewicht des Träger-Enzym-Produkts, an freien SH-Gruppen gebunden ist. Schwefelwasserstoff und Verbindungen mit 2 oder mehr Merkaptogruppen enthalten zwei (oder mehr) an Schwefel gebundene Wasserstoffatome, so daß nach ihrer Bindung an den

3

Träger auf jeden Fall wenigstens eine freie Merkaptogruppe übrig bleibt. Verbindungen mit zwei oder mehr SH-Gruppen sind besonders bevorzugt. Dazu gehören Dithioäthylenglykol, Dithiopropylenglykol oder Dithiobutylenglykol, 1,12-Dimerkaptododecan, Di-merkaptoessigester des Äthylenglykols, Dodecandiols, Neopentylglykols oder des 2,2'-Bis-(4-hydroxy-cyclohexyl)-propans, Trimethylolpropantri-merkaptopropionat, Pentaerythrit-tetra-merkaptoacetat, -tetra-merkaptopropionat, -tetra-merkaptocapronsäureester oder -tetra-merkaptoundecansäureester sowie Verbindungen mit weiteren funktionellen Gruppen, wie Dithioerythrit. Als Verbindungen mit je einer Amino- und einer Merkaptogruppe seien Merkaptoäthylamin und Cystein genannt. Verbindungen mit nur einer Merkapto- und einer oder mehr Hydroxylgruppen sind am wenigsten bevorzugt, da die Merkaptogruppen in der Regel leichter als die Hydroxylgruppen mit den Oxirangruppen des Trägers reagieren. Beispiele dieses Verbindungstyps sind Monothioäthylenglykol und Monothiopropylenglykol.

Es ist eine Vielzahl von wasserunlöslichen Trägermaterialien mit gegenüber Proteinen bindungsaktiven Oxirangruppen bekannt. Eine Übersicht über derartige Träger findet sich in O. Zaborsky, "Immobilized Enzymes", Cleveland 1973. Als wichtigste Trägermaterialien werden dort synthetische vernetzte Polymere auf Basis von Acrylamid, Maleinsäureanhydrid, Methacrylsäure, Styrol oder Polypeptiden genannt. Als natürliche Trägerstoffe werden Kohlenhydrate, wie Agarose, Cellulose, vernetztes Dextran oder Stärke erwähnt. Auch Glas und andere anorganische Stoffe kommen als Träger in Betracht. Die Zahl der Oxirangruppen soll groß genug sein, um wenigstens 10 mg, vorzugsweise 100 mg oder mehr Protein je Gramm Trägermaterial zu binden.

Oxirangruppen können schon bei der Herstellung eines vernetzten Acrylamidopolymerisats, vorzugsweise eines durch umgekehrte Suspensionspolymerisation erzeugten Perlpolymerisats, durch Mitverwendung glycidylgruppenhaltiger Comonomerer, wie Glycidyl-acrylat oder -methacrylat, eingebaut werden. In Trägerkörper mit Kohlenhydratstruktur, wie Cellulose, Agarose oder Dextran, können Glycidylgruppen z.B. durch Umsetzung mit Epichlorhydrin oder 1,4-Bis-(2,3-epoxypropyl)-butan eingeführt werden. Perlförmige Trägerstoffe mit Perldurchmessern von 5 bis 5000 $\mu$m sind wegen ihrer leichten Handhabbarkeit und ihrer Strömungsdurchlässigkeit besonders vorteilhaft.

Die Natur des zu bindenden Proteins richtet sich nach dem vorgesehenen Verwendungszweck des Verfahrensproduktes. Für Zwecke der Affinitätschromatographie wird ein Protein an den Träger gebunden, das in biospezifische Wechselwirkung zu einer Komponente eines aufzutrennenden Substratgemisches treten kann. Diese Komponente wird an dem trägergebundenen Protein vorübergehend festgehalten. Vorzugsweise werden Enzyme an den Träger gebunden, wie z.B. Penicillinacylase, Ribonuclease, Amylase oder Katalase. Im Sinne der Erfindung gebundene und stabilisierte Penicillinacylase hat besondere technische Bedeutung, weil erst dadurch eine wirtschaftliche Wiederverwendung dieses wertvollen Enzyms im technischen Maßstab möglich wird.

Einige Enzyme sind gegenüber Schwefelwasserstoff oder Merkaptanen empfindlich; sie eignen sich dann im allgemeinen nicht für das Verfahren der Erfindung Enzyme, die schon durch Schwefelwasserstoff oder freie Merkaptane stabilisiert oder reaktiviert werden, lassen sich durch das Verfahren der Erfindung in Enzymprodukte von erhöhter Stabilität überführen.

## Beispiel 1

a) *Herstellung von Oxiranacrylharzperlen*

In einem Rundkolben (6000 ml), versehen mit Thermometer, Impeller-Rührer, Rückflußkühler und N$_2$-Einleitungsrohr werden

1750 g n-Heptan
1100 g Perchloräthylen
2 g Polymerisat der Zusammensetzung
95% n-Butylmethacrylat
5% 2-Trimethylammoniumäthylmethacrylat-chlorid

vorgelegt. Unter Einleitung von Stickstoff wird bei ca. 55°C eine Mischung aus

791 g Formamid
60 g Methacrylamid
60 g Allylglycidäther
60 g Glycidylmethacrylat
120 g Methylen-bis-methacrylamid
6 g Benzoylperoxid

zugegeben und durch Rühren in der organischen Phase verteilt. Die Polymerisation wird durch Zugabe von 6 g Dimethylanilin beschleunigt. Nach 10-stündiger Polymerisation wird auf Raumtemperatur abgekühlt, die Perlen absetzen gelassen und die überstehende flüssige Phase abdekantiert. Die Perlen werden durch Zugabe von ca. 2000 ml Aceton entquollen, abgesaugt und zweimal mit Aceton nachgewaschen. Anschließend werden sie über Nacht bei Raumtemperatur im Vakuum getrocknet. Ausbeute: ca. 90% feine, leicht gelbl. Perlen.

b) *Immobilisierung der Penicillinacylase*

1,2 g Penicillinacylase (E.coli, spez. Aktivität 12,1 U/mg) werden in 40 ml 0,1 M Phosphatpuffer (pH 8, 0,02% NaN$_3$) gelöst und zu 10 g des Produkts (1a) gegeben. Man läßt bei 23°C 72 Stunden lang

# 0 000 486

stehen und wäscht anschließend dreimal mit jeweils 1000 ml 1 M wäßriger NaCl-Lösung und zweimal mit 0,05 M Phosphatpuffer (pH 7,5; 0,02% NAN₃). Ausbeute: 37 g Feuchtprodukt (nach Absaugen auf Glasfritte). Enzymatische Aktivität: 237 U/g Feuchtprodukt.

c) *Aktivitätsbestimmung*

2 g Penicillin-G (Kaliumsalz) werden in 90 ml Phosphatpuffer (pH 7,5; 0,05 M) gelöst und das Volumen mit diesem Puffer auf 100 ml gebracht.

20 ml dieser Substratlösung und 250 mg (Feuchtegewicht!) des Produktes (1b) werden in einem Titrierstand (TTA 3, Fa. Radiometer, Kopenhagen) bei 37°C thermostatisiert gerührt. Die durch Hydrolyse freiwerdende Phenylessigsäure wird bei pH 7,8 mit 1 N NaOH aus einer Autobürette (ABU 12, Fa. Radiometer, Kopenhagen) automatisch titriert, über pH-Meter und Titrator-(Typ 11, Fa. Radiometer, Kopenhagen) gesteuert. Simultan wird der NaOH-Verbrauch mit Hilfe eines Schreibers (Tritrigraph SBR 2c, Fa. Radiometer, Kopenhagen) registriert.

Die Aktivität wird aus dem linearen Anfangsbereich der Umsatzkurve graphisch ermittelt; 1 Enzymeinheit (U) entspricht 1 $\mu$Mol gespaltenem Substrat pro Minute.

d) *Aktivitätsverlust bei wiederholter Verwendung bei 95%*
   *Umsatz von Substratlösungen einer Konzentration von 5%*

2,0 g des Produktes (1b) und 20 ml einer 5%igen Substratlösung (5% Penicillin-G-K in 0,01 M Phosphatpuffer, pH 7,5 enthaltend 0,02% NaN₃) werden bei pH 7,5 und 37°C bis zu einem Substratumsatz von 95—98% gerührt. Apparatur und Registrierung der Umsatkurve wie unter (1c) beschrieben.

Dann werden die Enzymperlen auf einer Glasfritte abgesaugt und erneut gegen 20 ml Substratlösung eingesetzt. Diese Prozedur wird 20 mal hintereinander wiederholt.

Aktivitätsverlust nach 20 Verwendungen: 57%.

## Beispiel 2

Zu 2 g der trägergebundenen Penicillinacylase nach Beispiel 1(b) werden 10 ml einer wäßrigen Lösung von Dithioglykol (2% mit NaOH auf pH 7,5 eingestellt) gegeben. Man läßt das Gemisch bei 37°C 18 h lang stehen und wäscht mit Wasser (zehnmal jeweils 100 ml) und zweimal mit 0,05 M Phosphatpuffer (pH 7,5).

Ausbeute: 1,95 g Feuchtprodukt.

Das Produkt wird wie in Beispiel 1 (a) geprüft. Aktivitätsverlust nach 20 wiederholten Verwendungen: 42%.

## Beispiel 3

50 mg 1,4-Dithioerythrit werden in 1 ml H₂O gelöst und der pH-Wert durch Zugabe von NaOH auf 7,5 eingestellt. Die Lösung wird zu 2 g trägergebundenen Penicillinacylase nach Beispiel 1 (b) gegeben und das Gemisch wird 48 h lang bei 23°C stehen gelassen. Dann wird es mit Wasser (fünfmal) und Puffer (zweimal) gewaschen.

Ausbeute: 1,98 g.

Aktivitätsverlust nach 20 wiederholten Verwendungen, durchgeführt wie im Beispiel 1 (d): 35%.

## Beispiel 4

500 mg Dithioerythrit werden in 5 ml Wasser gelöst und durch NaOH-Zugabe wird ein pH von 7,8 eingestellt. Diese Lösung wird zu 2 g der trägergebundenen Penicillinacylase nach Beispiel 1 (b) gegeben und das Gemisch bei 23°C 48 h lang stehen gelassen. Dann wird es mit Wasser (fünfmal) und Puffer (zweimal) gewaschen.

Aktivitätsverlust nach 20 wiederholten Verwendungen, durchgeführt wie im Beispiel 1 (d): 11%.

## Patentansprüche

1. Verfahren zur Herstellung von stabilisierten trägergebundenen Proteinen durch Umsetzung eines Proteins in wäßriger Lösung und eines SH-Gruppen enthaltenden Stabilisators mit einem wasserunlöslichen Trägermaterial, das gegenüber Proteinen bindungsaktive Gruppen enthält, dadurch gekennzeichnet, daß ein merkaptogruppen-freies Trägermaterial eingesetzt wird, das als bindungsaktive Gruppen Oxirangruppen enthält, und daß man auf das an den Träger gebundene Protein Schwefelwasserstoff oder eine Verbindung mit einem Molekulargewicht unter 5000 mit wenigstens einer Merkaptogruppe und wenigstens einer weiteren Merkapto-, Hydroxy- oder primären oder sekundären Aminogruppe einwirken läßt.

2. Verfahren nach Anspruch 1, dadurch gekennzeichnet, daß ein Trägermaterial auf Basis eines Polyacrylamids oder von Kohlehydratderivaten verwendet wird.

3. Verfahren nach den Ansprüchen 1 und 2, dadurch gekenzeichnet, daß ein perlförmiges Trägermaterial mit einem Perldurchmesser zwischen 5 und 1000 $\mu$m auf Basis eines vernetzten Polyacrylamids mit Oxirangruppen eingesetzt wird.

4. Verfahren nach den Ansprüchen 1 bis 3, dadurch gekennzeichnet, daß man als Verbindung mit

**0 000 486**

wenigstens einer Merkaptogruppe eine aliphatische Verbindung mit 2 bis 8 C-Atomen einsetzt.

5. Verfahren nach den Ansprüchen 1 bis 4, dadurch gekennzeichnet, daß das gebundene Protein ein Enzym ist.

6. Verfahren nach Anspruch 5, dadurch gekennzeichnet, daß das gebundene Enzym Penicillin-acylase, Ribonuklease, Amylase oder Katalase ist.

7. Verfahren nach Anspruch 5, dadurch gekennzeichnet, daß das gebundene Enzym Penicillin-acylase ist.

8. Verfahren nach den Ansprüchen 1 bis 6, dadurch gekennzeichnet, daß wenigstens 0,3 Gew.-% (bezogen auf das Feuchtgewicht des Endprodukts) an freien Merkaptogruppen an das Trägermaterial angelagert werden.

## Revendications

1. Procédé pour la préparation de protéines stabilisées, fixées sur un support, par réaction d'une protéine en solution aqueuse et d'un stabilisant contenant des groupes SH avec une matière de support insoluble dans l'eau qui contient des groupes ayant, à l'égard des protéines, une action de fixation, caractérisé en ce qu'on met en oeuvre une matière de support exempte de groupes mercapto qui contient des groupes oxirane en tant que groupes ayant un pouvoir de fixation, et en ce qu'on fait agir, sur la protéine fixée sur le support, de l'acide sulfhydrique ou un composé ayant un poids moléculaire inférieur à 5000 et comportant au moins un groupe mercapto et au moins un autre groupe mercapto, un groupe hydroxy ou un groupe amino primaire ou secondaire.

2. Procédé selon la revendication 1, caractérisé en ce qu'on utilise une matière de support à base d'un polyacrylamide ou de dérivés d'hydrates de carbone.

3. Procédé selon la revendication 1 ou 2, caractérisé en ce qu'on met en oeuvre une matière de support en perles, ayant un diamètre des perles compris entre 5 et 1000 microns, à base d'un polyacrylamide ramifié comportant des groupes oxirane.

4. Procédé selon l'une quelconque des revendications 1 à 3, caractérisé en ce qu'on met en oeuvre, en tant que composé comportant au moins un groupe mercapto, un composé aliphatique à 2 à 8 atomes de carbone.

5. Procédé selon l'une quelconque des revendications 1 à 4, caractérisé en ce que la protéine fixée est un enzyme.

6. Procédé selon la revendication 5, caractérisé en ce que l'enzyme fixé est une pénicilline-acylase, une ribonucléase, un amylase ou une catalase.

7. Procédé selon la revendication 6, caractérisé en ce que l'enzyme fixé est une pénicilline-acylase.

8. Procédé selon l'une quelconque des revendications 1 à 7, caractérisé en ce qu'au moins 0,3% en poids (sur la base du poids humide du produit final) de groupes mercapto libres sont fixés par addition à la matière de support.

## Claims

1. Method of preparing stabilised carrier-bound proteins by reacting a protein in aqueous solution and a stabiliser containing SH groups with a water-insoluble carrier material which contains groups capable of bonding with proteins, characterised in that a carrier material free from mercapto groups is used which contains oxirane groups as the actively bonding groups, and in that the protein bound to the carrier is reacted with hydrogen sulphide or a compound with a molecular weight of less than 5000 with at least one mercapto group and at least one further mercapto, hydroxy or primary or secondary amino group.

2. Method according to claim 1, characterised in that a carrier material based on a polyacrylamide or carbohydrate derivatives is used.

3. Method according to claims 1 and 2, characterised in that a carrier material in the form of beads having a bead diameter of between 5 and 1000 $\mu$m based on a cross-linked polyacrylamide with oxirane groups is used.

4. Method according to claims 1 to 3, characterised in that an aliphatic compound with from 2 to 8 carbon atoms is used as the compound with at least one mercapto group.

5. Method according to claims 1 to 4, characterised in that the bound protein is an enzyme.

6. Method according to claim 5, characterised in that the bound enzyme is peniciliinacylase, ribonuclease, amylase or catalase.

7. Method according to claim 6, characterised in that the bound enzyme is penicillinacylase.

8. Method according to claims 1 to 7, characterised in that at least 0.3% by weight (based on the moist weight of the end product) of free mercapto groups are taken up by the carrier material.